Europäisches Patentamt

(19) European Patent Office

Office européen des brevets

(11) Veröffentlichungsnummer: **0 015 513**
**B2**

(12) **NEUE EUROPÄISCHE PATENTSCHRIFT**

(45) Veröffentlichungstag der neuen Patentschrift:
**21.08.85**

(51) Int. Cl.⁴: **C 07 C 11/09**, C 07 C 7/148,
C 07 C 41/01, C 07 C 43/04

(21) Anmeldenummer: **80101005.9**

(22) Anmeldetag: **29.02.80**

(54) Verfahren zur Gewinnung von Isobuten aus Isobuten enthaltenden C4-Kohlenwasserstoffgemischen.

(30) Priorität: **05.03.79 DE 2908426**

(43) Veröffentlichungstag der Anmeldung:
**17.09.80 Patentblatt 80/19**

(45) Bekanntmachung des Hinweises auf die Patenterteilung:
**10.11.82 Patentblatt 82/45**

(45) Bekanntmachung des Hinweises auf die Entscheidung
über den Einspruch:
**21.08.85 Patentblatt 85/34**

(84) Benannte Vertragsstaaten:
**AT BE CH DE FR GB IT NL SE**

(73) Patentinhaber: **BASF Aktiengesellschaft,
Carl-Bosch-Strasse 38, D-6700 Ludwigshafen (DE)**

(72) Erfinder: **Lindner, Alfred, Dr., Ringstrasse 22,
D-6712 Bobenheim-Roxheim 1 (DE)**
Erfinder: **Volkamer, Klaus, Dr., Heidelberger Ring 21,
D-6710 Frankenthal (DE)**
Erfinder: **Wagner, Ulrich, Dr., Knospstrasse 7,
D-6703 Limburgerhof (DE)**

(56) Entgegenhaltungen:
**DE - A - 2 521 673
DE - A - 2 521 964
DE - A - 2 629 769
DE - A - 2 802 198
DE - A - 2 802 199
DE - B - 1 216 865
DE - B - 1 934 422
US - A - 3 170 000
US - A - 3 634 535**

**SRI Report NO 78-1-3 vom Dezember 1978 Process
Economics Review, SRI International**

## Beschreibung

Die vorliegende Erfindung betrifft ein Verfahren zur Gewinnung von Isobuten aus Isobuten enthaltenden $C_4$-Kohlenwasserstoffgemischen durch Umsetzung des Gemisches mit einem primären $C_3$- oder $C_4$-Alkohol und Zerlegung des gebildeten tertiären Äthers bei erhöhten Temperaturen.

Es ist bereits bekannt, Isobuten durch Anwendung von Schwefelsäure-Extraktionsverfahren aus $C_4$-Kohlenwasserstoffgemischen zu gewinnen. Bei diesen Schwefelsäure-Extraktionsverfahren muß Schwefelsäure hoher Konzentration verwendet werden, und infolgedessen ist die Verwendung kostspieliger Materialien für die Ausrüstung erforderlich. Da weiterhin Nebenreaktionen des Isobutens, beispielsweise Dimerisation, Polymerisation, Hydratation und dergl. während der Extraktion erfolgen, ist das Schwefelsäure-Extraktionsverfahren hinsichtlich Ausbeute und Qualität der Produkte nicht stets zufriedenstellend.

Es sind weiter, z. B. aus US-A 3 170 000, DE-A2-1 216 865 und US-A1-3 634 535 Verfahren zur Gewinnung von Isobuten bekannt, bei denen in einer ersten Stufe Isobuten mit Methanol umgesetzt wird und der gebildete Methyl-tert.-butyläther in einer zweiten Stufe in Methanol und Isobuten zerlegt wird. Die bekannten Verfahren haben jedoch den Nachteil, daß bei der Verwendung von Isobuten enthaltenden $C_4$-Kohlenwasserstoffgemischen als Ausgangsstoff Methanol mit den $C_4$-Kohlenwasserstoffen azeotrope Gemische bildet, so daß die Abtrennung des Methanols aus dem nach der Verätherungsstufe erhaltenen Reaktionsgemisch sehr erschwert ist und beispielsweise eine aufwenige Wasserwäsche eingeschaltet werden muß. Ein weiterer Nachteil der bekannten Verfahren besteht darin, daß die Ausbeute bei der Umsetzung des Isobutens mit einem primären Alkohol zum tertiären Äther nicht zufriedenstellend ist (vgl. besonders US-A-1-3 634 535, Spalte 6 mit Propanol als primärer Alkohol) und daher bei der Verwendung von Isobuten enthaltenden $C_4$-Kohlenwasserstoffgemischen als Ausgangsstoff aus dem nach der Verätherung erhaltenen Reaktionsgemisch ein $C_4$-Kohlenwasserstoffgemisch aus den nicht umgesetzten Kohlenwasserstoffen mit einem unbefriedigend hohen Isobuten-Gehalt erhalten wird.

Aus der EP-A-2-003 305 ist außerdem ein Verfahren zur Gewinnung von Isobuten aus Isobuten enthaltenden $C_4$-Kohlenwasserstoffgemischen durch Umsetzung des Gemisches in einer Verätherungsstufe mit einem primären $C_3$- oder $C_4$-Alkohol und Zerlegung des gebildeten tertiären Äthers bei erhöhten Temperaturen bekannt, wobei für die Austrittstemperaturen des Reaktionsgemisches aus der Reaktionszone der Verätherung bestimmte Temperaturen angewendet werden.

Es wurde nun ein vorteilhaftes Verfahren gefunden zur Gewinnung von Isobuten aus Isobuten enthaltenden $C_4$-Kohlenwasserstoffgemischen durch Umsetzung des Gemisches mit einem primären $C_3$- oder $C_4$-Alkohol in Gegenwart eines Ionenaustauschers in der Wasserstofform, wobei dieser in einem Festbett angeordnet ist, Destillation des bei der Verätherung erhaltenen Reaktionsgemisches, wobei man als Kopfprodukt das $C_4$-Kohlenwasserstoff-Raffinat und als Bodenprodukt den gebildeten tertiären Äther, gegebenenfalls noch mit überschüssigem primärem $C_3$- oder $C_4$-Alkohol abzieht, Zerlegung des Bodenproduktes in einer zweiten, einen sauren Katalysator enthaltenden Reaktionszone bei erhöhter Temperatur in Isobuten und primären $C_3$- oder $C_4$-Alkohol, Destillation des Gemisches aus Isobuten und primärem $C_3$- oder $C_4$-Alkohol, wobei also Kopfprodukt Isobuten und als Bodenprodukt der primäre $C_3$- oder $C_4$-Alkohol abgezogen werden, und Rückführung des erhaltenen primären $C_3$- oder $C_4$-Alkohols in die Reaktionszone für die Verätherung, welches dadurch gekennzeichnet ist, daß die Austrittstemperatur des Reaktionsgemisches aus der Verätherungsstufe für die Bildung des tertiären Äthers 3 bis 30° C tiefer liegt als die mittlere Temperatur in der Verätherungsstufe und daß die Austrittstemperatur des Reaktionsgemisches aus der Stufe für die Zerlegung des gebildeten tertiären Äthers 3 bis 30° C höher liegt als die mittlere Temperatur in der Zerlegungsstufe.

Nach dem neuen Verfahren läßt sich aus dem nach der Verätherungsstufe erhaltenen Reaktionsgemisch ein praktisch alkoholfreies $C_4$-Kohlenwasserstoff-Raffinat durch einfache Destillation ohne Einschaltung einer Wasserwäsche abtrennen, da nicht umgesetzter primärer $C_3$- oder $C_4$-Alkohol überraschenderweise keine Azeotrope mit den $C_4$-Kohlenwasserstoffen bildet. Im allgemeinen beträgt die Konzentration an $C_3$- oder $C_4$-Alkohol im $C_4$-Kohlenwasserstoff-Raffinat höchstens 50 Gew.-ppm, vorzugsweise höchstens 20 Gew.-ppm, insbesondere höchstens 5 Gew.-ppm. Für das erfindungsgemäße Verfahren ist daher ein wesentlich geringerer Trennaufwand erforderlich als bei dem bekannten Verfahren. Ein weiterer Vorteil des erfindungsgemäßen Verfahrens besteht darin, daß in einfacher Weise auch bei Einsatz eines kleinen Reaktors der Umsatz des im Ausgangs-$C_4$-Kohlenwasserstoffgemisch enthaltenen Isobutens mit dem primären Alkohol zum tertiären Äther merklich erhöht wird, so daß aus dem nach der Verätherung erhaltenen Reaktionsgemisch ein $C_4$-Kohlenwasserstoff-Raffinat aus den nicht umgesetzten Kohlenwasserstoffen mit einem deutlich erniedrigten Isobutengehalt abgetrennt werden kann. Der Isobutenverlust bei der Umsetzung zum tertiären Äther kann daher erheblich gesenkt werden. Das erhaltene, weitgehend isobutenfreie $C_4$-Kohlenwasserstoff-Raffinat ist hervorragend geeignet für bestimmte Verwendungszwecke, bei denen ein möglichst isobuten armes $C_4$-Kohlenwasserstoff Raffinat benötigt

wird, z. B. als Ausgangsstoff für die Herstellung von sek.-Butanol, Methyläthylketon, Buten-1, Octenen oder Maleinsäureanhydrid.

Es ist weiter ein Vorteil des neuen Verfahrens, daß als C4-Kohlenwasserstoffgemisch unmittelbar butadienhaltige C4-Fraktionen, wie sie z. B. aus Äthylenanlagen oder Butan-Buten-Dehydrier-Anlagen erhalten werden, verwendet werden können. Eine vorherige Butadien-Extraktion aus der C4-Fraktion ist nicht erforderlich.

Das Isobuten wird nach dem Verfahren der Erfindung in hoher Ausbeute erhalten. Es war überraschend, daß diese hohe Ausbeute mit einem höheren Alkohol wie einem primären C3- oder C4-Alkohol möglich war, da aus der US-PS 3 170 000 insbesondere Tabelle 1 bekannt ist, daß bei der Umsetzung von C5-Kohlenwasserstoffgemischen mit Alkoholen bei Verwendung von C3- oder C4-Alkoholen wesentlich schlechtere Ausbeuten an tertiärem Äther erhalten werden als bei der Verwendung von Äthanol oder Methanol. Nach dem erfindungsgemäßen Verfahren wird dagegen in der Verätherungsreaktion das im C4-Kohlenwasserstoffgemisch enthaltene Isobuten im allgemeinen zu mindestens 90%, vorzugsweise zu mindestens 95% zum C3- oder C4-Alkyl-tert.-butyläther umgesetzt.

Für das erfindungsgemäße Verfahren geeignete, Isobuten enthaltende C4-Kohlenwasserstoffgemische werden beispielsweise beim thermischen oder katalytischen Cracken von Erdölprodukten, bei der Herstellung von Äthylen durch Pyrolyse von verflüssigtem Petroleumgas (LPG), Leichtbenzin (Naphtha), Gasöl oder dergl., oder bei der katalytischen Dehydrierung von n-Butan und/oder n-Buten erhalten. Diese C4-Kohlenwasserstoffgemische enthalten in der Regel neben dem Isobuten olefinische und paraffinische C4-Kohlenwasserstoffe und können darüber hinaus noch Butadien, z. B. in einer Menge von bis zu 70 Gewichtsprozent, und höhere Acetylene, wie Butin-1 und Butenin enthalten. Butadien enthaltende C4-Kohlenwasserstoffgemische können als solche oder nach vorheriger Abtrennung des Butadiens aus dem C4-Kohlenwasserstoffgemisch, z. B. durch Butadien-Extraktion unter Verwendung eines selektiven Lösungsmittels, verwendet werden. Die C4-Kohlenwasserstoffgemische können außerdem noch C3-Kohlenwasserstoffe wie Propan, Propen, Propin, z. B. bis zu 10 Gewichtsprozent, enthalten. Die C4-Kohlenwasserstoffgemische weisen im allgemeinen einen Isobutengehalt von 5 bis 95 Gewichtsprozent, vorzugsweise 10 bis 90 Gewichtsprozent, insbesondere 20 bis 70 Gewichtsprozent, auf. Vorzugsweise werden solche C4-Kohlenwasserstoffgemische verwendet, die neben Isobuten n-Butan, Isobutan, Buten-1, trans-Buten-2 und cis-Buten-2 und gegebenenfalls Butadien-1,3 enthalten.

Als erfindungsgemäß zu verwendende primäre C3- oder C4-Alkohole (d. h. Alkohole mit 3 oder 4 Kohlenstoffatomen) werden n-Propanol, n-Butanol oder Isobutanol, vorzugsweise n-Propanol oder Isobutanol, und insbesondere Isobutanol eingesetzt. Die Alkohole werden z. B. als technische Produkte üblicher Reinheit, beispielsweise mit einer Reinheit von mindestens 95%, vorzugsweise mindestens 98%, verwendet.

Als saure Kondensationsmittel für die Verätherungsstufe kommen Ionenaustauscher in der Wasserstofform in Betracht. Geeignete Ionenaustauscher sind beispielsweise sulfonierte Kohlen, Zeolithe, sulfonierte Phenol-Formaldehyd-Harze, sulfonierte, von Cumaron-Inden-Kondensationsprodukten abgeleitete Harze sowie insbesondere sulfonierte Polystyrol-Harze wie kernsulfonierte vernetzte Styrol-Divinylbenzol-Copolymerisate; deren Menge beträgt im allgemeinen 0,01 bis 1,1 Schüttvolumen pro 1 Reaktorvolumen.

Für die Verätherungsstufe können als Reaktoren z. B. Strömungsrohrreaktoren, beispielsweise ein Schlaufenreaktor, verwendet werden. Die Reaktoren werden als Festbettreaktoren eingesetzt.

Es kann vorteilhaft sein, in der Verätherungsstufe mehrere Reaktionszonen, im allgemeinen 2 bis 10 Reaktionszonen, vorzugsweise 2 bis 6 Reaktionszonen, hintereinanderzuschalten. Bei der Verwendung von mehreren hintereinandergeschalteten Reaktionszonen kann es zweckmäßig sein, einen Teilstrom aus einer Reaktionszone oder je einen Teilstrom aus mehreren oder allen Reaktionszonen abzuzweigen und in den Hauptstrom vor einer der vorhergehenden Reaktionszonen zurückzuführen.

Für die Verätherung wird das Kohlenwasserstoffgemisch mit dem primären C3- oder C4-Alkohol in Gegenwart des Ionenaustauschers in der Wasserstofform im allgemeinen bei Temperaturen von 20 bis 120°C umgesetzt. Die Maximaltemperatur in der Verätherungsstufe liegt hierbei im allgemeinen zwischen 50 und 120°C, vorzugsweise zwischen 70 und 120°C. Die Austrittstemperatur aus der Verätherungsstufe liegt im allgemeinen zwischen 20 und 70°C, vorzugsweise zwischen 30 und 50°C.

Das erfindungsgemäße Verfahren wird in der Weise durchgeführt, daß die Austrittstemperatur des Reaktionsgemisches aus der Verätherungsstufe für die Bildung des tertiären Äthers 3 bis 30°C, vorzugsweise 5 bis 20°C, insbesondere 7 bis 15°C, tiefer liegt als die mittlere Temperatur in der Verätherungsstufe.

Die mittlere Temperatur in der Verätherungsstufe wird zweckmäßig nach folgender Gleichung ermittelt:

$$T_m = \frac{1}{V_G} \int_{v=0}^{v=V_G} T dv. \tag{1}$$

In der Gleichung (1) bedeuten:

$T_m$ = mittlere Temperatur in der Verätherungsstufe

$T$ = Temperatur am Volumenelement v

$v$ = Volumen

$V_G$ = Gesamtvolumen der Verätherungsstufe

Hieraus ergibt sich, daß, falls für die Verätherungsstufe mehrere Strömungsrohre verwendet werden, die mittlere Temperatur in der Verätherungsstufe zweckmäßig nach folgender Gleichung ermittelt wird:

$$T_m = \frac{1}{V_s} \sum_{i=0}^{i=S} \int_{v=0}^{v=V_i} T_i dv . \qquad (2)$$

In der Gleichung (2) bedeuten:

$T_m$  mittlere Temperatur in der Verätherungsstufe

$T_i$  Temperatur im Strömungsrohr i am Volumen v

$V_i$  Volumen des i-ten Strömungsrohres

S  Anzahl der in der Verätherungsstufe hintereinandergeschalteten Strömungsrohre

$V_S$  Gesamtvolumen der Strömungsrohre.

Bei Verwendung einer einzigen Reaktionszone für die Verätherungsstufe lassen sich die mittlere Temperatur in dieser Stufe und die Austrittstemperatur aus dieser Stufe z. B. durch direkte Kühlung der Reaktionszone durch Einstellen von Kühlmittelmenge, Kühlmitteltemperatur und Kühlmittelfließrichtung, z. B. Gegenstromkühlung, verändern. Enthält die Verätherungsstufe mehrere Reaktionszonen, so können die mittlere Temperatur in der Verätherungsstufe und die Austrittstemperatur aus der Verätherungsstufe · z. B. durch unterschiedliches Kühlen einer oder mehrerer Reaktionszonen und oder durch Zwischenkühlung des Stromes zwischen mehreren Reaktionszonen, beispielsweise zwischen 2 Reaktionszonen, verändern. Die mittlere Temperatur und die Austrittstemperatur können weiter z. B. durch Variierung der Eintrittstemperatur der Reaktionsmischung in die Verätherungsstufe und gegebenenfalls durch Rückführungen von Teilströmen und/oder durch Quenchen des Stromes oder von Teilströmen in die Verätherungsstufe reguliert werden.

Die erfindungsgemäße Verätherung kann bei Normaldruck erfolgen. Es ist jedoch zweckmäßig, einen geringen Überdruck, z. B. Drücke von 1,01 bis 30 bar, insbesondere 2 bis 20 bar, anzuwenden. Das Isobuten enthaltende C4-Kohlenwasserstoffgemisch kann dabei je nach Druck und Temperatur für die Umsetzung flüssig oder gasförmig eingesetzt werden. Vorzugsweise werden flüssige Isobuten enthaltende C4-Kohlenwasserstoffgemische eingesetzt. Die Verätherung kann diskontinuierlich durchgeführt werden. Die Reaktionszeiten liegen bei diskontinuierlicher Arbeitsweise im allgemeinen zwischen 1 Minute und 5 Stunden. Vorzugsweise wird die Verätherung jedoch kontinuierlich durchgeführt, wobei das Verhältnis aus Reaktorvolumen in 1 und dem Durchsatz in 1/h im allgemeinen 0,01 bis 5 Stunden, vorzugsweise 0,3 bis 1 Stunde, beträgt.

Das Gewichtsverhältnis von primärem C3- oder C4-Alkohol zu dem im C4-Kohlenwasserstoffgemisch enthaltenen Isobuten beträgt bei der Verätherung im allgemeinen 100 : 1 bis 1 : 1, vorzugsweise 20 : 1 bis 1,2 : 1, insbesondere 4 : 1 bis 1,3 : 1.

Das nach der Verätherung erhaltene Reaktionsgemisch, welches in der Regel noch im Überschuß zur Verätherung zugesetzten primären C3- oder C4-Alkohol enthält, wird durch konventionelle Destillation ohne Einschaltung einer Wasserwäsche aufgetrennt, wobei als Kopfprodukt ein weitgehend isobutenfreies C4-Kohlenwasserstoffgemisch-Raffinat erhalten wird, welches im allgemeinen einen Isobuten-Gehalt von höchstens 5 Gewichtsprozent, vorzugsweise höchstens 2,5 Gewichtsprozent, insbesondere höchstens 1,5 Gewichtsprozent, aufweist. Weiter enthält das Kopfprodukt der Destillation im allgemeinen höchstens 0,5 Gew.-%, vorzugsweise höchstens 0,02 Gew.-%, insbesondere höchstens 0,005 Gew.-% des primären C3- oder C4- Alkohols sowie im allgemeinen höchstens 0,005 Gew.-%, vorzugsweise höchstens 0,001 Gew.-%, insbesondere höchstens 0,0005 Gew.-% des tertiären Äthers.

Als Bodenprodukt der Destillation des nach der Verätherung erhaltenen Reaktionsgemisches wird der gegebenenfalls noch überschüssigen primären C3- oder C4-Alkohol enthaltende tertiäre Äther erhalten.

Der erhaltene tertiäre Äther wird anschließend in der zweiten Stufe des Verfahrens in Gegenwart eines sauren Katalysators bei erhöhten Temperaturen in Isobuten und primären C3- oder C4-Alkohol zerlegt. Als Ausgangsprodukt für die Zerlegungsstufe kann tertiärer Äther, der praktisch frei von primärem C3- oder C4-Alkohol ist, verwendet werden, der beispielsweise durch Verwendung einer Menge an primärem C3- oder C4-Alkohol bei der Verätherung, die höchstens der stöchiometrisch erforderlichen Alkoholmenge entspricht, oder durch Abtrennung, z. B. durch Abdestillieren, von überschüssig zugesetztem primärem C3- oder C4-Alkohol aus dem nach der Destillation des Reaktionsgemischs der Verätherung erhaltenen Bodenprodukt erhalten worden ist. Vorzugsweise wird der nach der destillativen Abtrennung des C4-Kohlenwasserstoffgemisch-Raffinats als Bodenprodukt erhaltene tertiäre Äther ohne weitere Abtrennung von gegebenenfalls vorhandenem überschüssigem C3- oder C4-Alkohol für die Zerlegung eingesetzt. Es ist jedoch auch möglich, nur einen Teil des überschüssigen C3- oder C4-Alkohols abzutrennen.

Für die Zerlegung wird der tertiäre Äther verdampft und mit dem sauren Katalysator in der Dampfphase kontaktiert. Als saure Katalysatoren kommen beispielsweise Ionenaustauscher in der Wasserstofform, wie sulfonierte Kohlen, Zeolithe, sulfonierte Phenol-Formaldehydharze, sulfonierte, von Cumaron-Inden-Kondensationsprodukte abgeleitete Harze sowie insbesondere sulfonierte Polystyrolharze wie kernsulfonierte, vernetzte Styrol-Divinylbenzol-Copolymerisate in Betracht.

Weiter sind feste Phosphorsäurekatalysatoren, die Mono- oder vorzugsweise Polyphosphorsäure auf einem festen Trägermaterial enthalten, vorteilhaft geeignet. Geeignete Trägermaterialien für die Phosphorsäurekatalysatoren sind z. B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfat, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z. B. Kieselgel, Aktivkohle, Aluminiumoxid oder Bims.

Weiter kommen Kieselgel oder Aluminiumoxid alleine als Katalysatoren für die Zerlegung in Betracht.

In einer weiteren Ausführungsform des erfindungsgemäßen Verfahrens wird für die Zerlegung als saurer Katalysator ein Metallphosphat, insbesondere ein Metallhydrogenphosphat, verwendet. Diese Phosphate können Phosphorsäure auch im Überschuß, der über die stöchiometrische Zusammensetzung der sauren Metallphosphate hinausgeht, enthalten, z. B. in einem Überschuß bis zu 65%, vorzugsweise bis zu 20%, insbesondere bis zu 10%. Als derartige Metallphosphate können beispielsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermaterialien sind beispielsweise Aluminiumoxid, Kieselsäure, Aktivkohle, Zinkoxid.

Die Menge des sauren Katalysators beträgt im allgemeinen etwa 0,01 bis 1 kg, vorzugsweise etwa 0,03 bis 0,3 kg je 1 kg/h Durchsatz des tertiären Äthers durch den Reaktor. Vorzugsweise werden für die Zerlegung des tertiären Äthers Festbettreaktoren verwendet.

Die Zerlegungstemperatur des tertiären Äthers variiert in Abhängigkeit von der Art des sauren Katalysators und der Kontaktzeit, liegt jedoch im allgemeinen bei Temperaturen von 50 bis 350°C, vorzugsweise 80 bis 300°C, insbesondere 100 bis 250°C. Bei der Verwendung von Metallphosphaten oder Phosphorsäurekatalysatoren als Zerlegungskatalysatoren werden im allgemeinen Temperaturen von 80 bis 350°C, vorzugsweise 90 bis 260°C, insbesondere 100 bis 210°C, angewendet, wobei es vorteilhaft sein kann, Austrittstemperaturen des Reaktionsgemisches aus der Zerlegungsstufe von 260°C, vorzugsweise 210°C, nicht zu überschreiten.

Für die Zerlegung des tertiären Äthers wird eine Austrittstemperatur des Reaktionsgemisches aus der Zerlegungsstufe eingehalten, die 3 bis 30°C, vorzugsweise 5 bis 20°C, insbesondere 7 bis 15°C höher liegt als die mittlere Temperatur in der Zerlegungsstufe. Die mittlere Temperatur in der Zerlegungsstufe wird zweckmäßig unter entsprechender Anwendung der vorstehend beschriebenen Formeln (1) oder (2) ermittelt. Die mittlere Tempeeratur in der Zerlegungsstufe und die Austrittstemperatur aus der Zerlegungsstufe können z. B. durch Zuführung unterschiedlicher Wärmemengen an den einzelnen Orten der Zerlegungsstufe eingestellt werden.

Die Kontaktzeit des verdampften tertiären Äthers beträgt zweckmäßig 0,1 bis 20 Sekunden, vorzugsweise 1 bis 10 Sekunden.

Die Zerlegung des tertiären Äthers kann bei Normaldruck erfolgen. Es ist jedoch auch möglich, Überdruck anzuwenden, z. B. Drücke bis zu 30 bar, vorzugsweise bis zu 20 bar, insbesondere Drücke von 1 bis 10 bar. Die Zerlegung kann jedoch auch bei vermindertem Druck durchgeführt werden.

Die Zerlegung wird im allgemeinen kontinuierlich durchgeführt. Von dem bei der Zerlegung erhaltenen Reaktionsgemisch, das als Reaktionsprodukte Isobuten und primären $C_3$- oder $C_4$-Alkohol enthält, wird das Isobuten durch Destillation abgetrennt. Dabei wird Isobuten im allgemeinen als Kopfprodukt der Destillation ohne Einschaltung einer Wasserwäsche mit einem Gehalt an primärem $C_3$- oder $C_4$-Alkohol von höchstens 500 Gew.-ppm, vorzugsweise höchstens 100 Gew.-ppm, abgezogen. Zweckmäßig weist das Isobutenprodukt eine Reinheit von mindestens 99,3 Gew.-%, vorzugsweise mindestens 99,5 Gew.-% auf, wobei jedoch ohne besonderen Aufwand auch Reinheiten von mehr als 99,8 Gew.-% erhalten werden können. Das verbleibende Produkt, das im wesentlichen den primären $C_3$- oder $C_4$-Alkohol enthält, wird zweckmäßig zur Verätherungsstufe zurückgeführt.

Bei kontinuierlicher Arbeitsweise und Rückführung des nach der Zerlegung des tertiären Äthers und anschließender Aufarbeitung des Reaktionsgemisches erhaltenen primären $C_3$- oder $C_4$-Alkohols in die Verätherungszone kann es bei dem neuen Verfahren zweckmäßig sein, bei der Verwendung von Isobutanol als $C_4$-Alkohol aus dem Isobutanolstrom zur Entfernung von eventuell angereicherten Verunreinigungen einen Isobutanolteilstrom abzuzweigen. Zweckmäßig wird aus dem Isobutanolstrom ein Teilstrom abgezweigt, der 0,1 bis 10 Gewichtsprozent, vorzugsweise 0,5 bis 5 Gewichtsprozent des Isobutanolstromes beträgt. In einer vorteilhaften Ausführungsform des Verfahrens wird der Isobutanolteilstrom in an sich bekannter Weise in Gegenwart eines Dehydratisierungskatalysators zu Isobuten dehydratisiert, wodurch die Ausbeute an Isobuten anders als bei den bekannten Verfahren zusätzlich erhöht wird.

Zweckmäßig wird die Dehydratisierung in der Gasphase an einem Katalysator durchgeführt. Geeignete Katalysatoren sind z. B. Kieselgel, Thoriumoxid, Titan(IV)oxid und insbesondere Aluminiumoxid. Im allgemeinen werden für die Dehydratisierung Temperaturen von 250 bis 450°C, vorzugsweise 300 bis 400°C, angewendet.

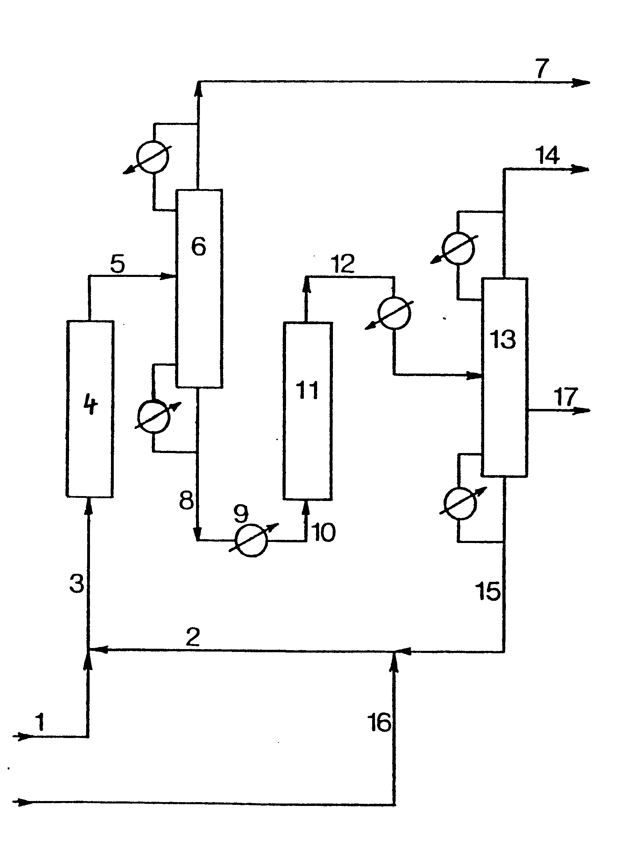

In der Figur wird eine beispielhafte Ausführungsform des erfindungsgemäßen Verfahrens schematisch erläutert. Das Isobuten enthaltende $C_4$-Kohlenwasserstoffgemisch (durch Leitung 1) und der primäre $C_3$- oder $C_4$-Alkohol (durch Leitung 2) werden vermischt, und die erhaltene Mischung wird durch Leitung 3 der Verätherungsstufe 4 zugeführt, die aus mehreren, z. B. 3 bis 5, hintereinandergeschalteten Reaktoren besteht, in denen sich der Ionenaustauscher befindet. Die Reaktoren sind als Festbettreaktor, z. B. als Strömungsrohr oder Schlaufenreaktor oder Kombination der beiden Reaktortypen ausgestaltet. In den hintereinandergeschalteten Reaktoren werden die Reaktionstemperaturen beispielsweise in der Weise eingestellt, daß der erste Reaktor, dem das Gemisch der Ausgangsstoffe zunächst zugeführt wird, die höchste Reaktionstemperatur aufweist und in den nachgeschalteten Reaktoren jeweils niedrigere Temperaturen eingestellt werden als in dem jeweils vorgeschalteten Reaktor. Die niedrigste Reaktionstemperatur weist dementsprechend der letzte der hintereinandergeschalteten Reaktoren auf, aus dem das Reaktionsgemisch nach beendeter Umsetzung über Leitung 5 abgezogen und einer ersten Destillationskolonne 6 zugeführt wird. Am Kopf der Destillationskolonne wird weitgehend isobutenfreies $C_4$-Kohlenwasserstoffgemisch (Raffinat) durch Leitung 7 abgezogen. Der als Bodenprodukt der Destillationskolonne 6 erhaltene tertiäre Äther, der gegebenenfalls noch im Überschuß zugesetzten primären $C_3$- oder $C_4$-Alkohol enthält, wird über Leitung 8 zunächst dem Verdampfer 9 zugeführt und nach der Verdampfung über Leitung 10 in die Zerlegungsstufe 11 eingeleitet, in der sich der saure Katalysator befindet. Für die Zerlegungsstufe 11 werden im allgemeinen Festbettreaktoren verwendet. Es können jedoch auch andere Reaktorentypen, z. B. Wirbelbettreaktoren, eingesetzt werden. Das aus der Zerlegungsstufe 11 abgezogene Gemisch aus Isobuten und primärem $C_3$- oder $C_4$-Alkohol wird über Leitung 12 in die Destillationskolonne 13 geleitet, in der als Kopfprodukt hochreines Isobuten erhalten wird, welches über Leitung 14 abgezogen wird. Der als Bodenprodukt erhaltene primäre $C_3$- oder $C_4$-Alkohol wird über Leitung 15 und 2, gegebenenfalls nach ergänzender Zugabe von primärem $C_3$- oder $C_4$-Alkohol über Leitung 16, dem Reaktor 4 für die Verätherung wieder zugeführt. Über Leitung 17 wird zweckmäßig ein kleiner, primären $C_3$- oder $C_4$-Alkohol enthaltender Teilstrom zur Ausschleusung von eventuell gebildeten Verunreinigungen wie Diisobutyläther, Diisobuten, Triisobuten, abgezogen. Dieser Teilstrom kann bei der Verwendung von Isobutanol als $C_4$-Alkohol einem Dehydratisierungsreaktor zugeführt werden, in dem zusätzlich Isobuten erhalten wird.

Nach dem erfindungsgemäßen Verfahren wird ein hochreines Isobuten erhalten, welches insbesondere für die Herstellung von hochmolekularen Polymeren des Isobutens geeignet ist.

Die folgenden Beispiele veranschaulichen die Erfindung.

### Beispiel

Die Verätherung wurde unter Verwendung eines $C_4$-Kohlenwasserstoffgemisches durchgeführt, welches den Rest (Raffinat I) einer aus einer Äthylenanlage erhaltenen $C_4$-Fraktion darstellte, aus der das Butadien extrahiert worden war. Die Zusammensetzung des $C_4$-Kohlenwasserstoffgemisches nach der Butadien-Extraktion war wie folgt:

| | |
|---|---|
| Isobutan | 1,9 Vol.-% |
| n-Butan | 8,1 Vol.-% |
| Isobuten | 46,0 Vol.-% |
| Buten-1 | 26,7 Vol.-% |
| trans-Buten-2 | 10,1 Vol.-% |
| cis-Buten-2 | 7,0 Vol.-% |
| Butadien-1,3 | 0,2 Vol.-% |

Eine Mischung aus 2000 g je Stunde dieses $C_4$-Kohlenwasserstoffgemisches und 2150 g je Stunde technisches Isobutanol wurde in die Verätherungsstufe eingeleitet, die aus drei hintereinandergeschalteten rohrförmigen Reaktoren bestand, die mit einem sulfonierten Polystyroldivinylbenzol-Harz in der Wasserstofform (®Lewatit SPC 118) gefüllt waren, wobei der erste Reaktor auf eine Temperatur von $T_1 = 80°C$, der zweite Reaktor auf eine Temperatur von $T_2 = 60°C$ und der dritte Reaktor auf eine Temperatur von $T_3 = 40°C$ thermostatisiert wurde.

Das Verhältnis der Volumina V der Reaktoren betrug $V_1 : V_2 : V_3 = 1 : 1,4 : 4$, die Verweilzeit, bezogen auf das gesamte leere Reaktorvolumen, 320 s. Die mittlere Temperatur in der Verätherungsstufe betrug 50,6°C. Das erhaltene Reaktionsgemisch wurde einer Destillationskolonne zugeführt, wobei am Kopf der Destillationskolonne ein Strom der nicht umgesetzten Kohlenwasserstoffe (Raffinat II) abgezogen wurde, der Isobutan, n-Butan, Buten-1, Buten-2-trans, Buten-2-cis und Butadien-1,3 enthielt. Das Raffinat II wies einen Isobuten-Gehalt von weniger als 2 Gew.-% auf. Es war praktisch isobutanolfrei und konnte daher ohne weitere Reinigungsoperationen, z. B. ohne Einschaltung einer Wasserwäsche, unmittelbar als Ausgangsstoff für weitere Reaktionen verwendet werden. Das Sumpfprodukt der Destillationskolonne bestand aus Isobutyl-tert.-butyläther, der noch im Überschuß zugesetztes Isobutanol enthielt.

### Vergleichsbeispiel

Die Verätherung wurde wie in Beispiel 1 beschrieben durchgeführt, wobei jedoch die drei Reaktoren auf die jeweils gleiche Temperatur von 50,6°C, die der mittleren Temperatur in Beispiel 1 entsprach, gehalten wurden. Das am Kopf der Destillationskolonne erhaltene Raffinat II wies einen Isobutengehalt von 3,0 Gew.-% auf. Bei Thermostatisierung der drei Reaktoren auf $T_1$ = 40°C, $T_2$ = 60°C und $T_3$ = 80°C enthielt das erhaltene Raffinat II sogar mehr als 3,4 Gew.-% Isobuten.

Das Sumpfprodukt der Destillation aus dem Beispiel, das Isobutyl-tert.-Butyläther und das im Überschuß zugesetzte Isobutanol enthielt, wurde verdampft und in einen erhitzten rohrförmigen Reaktor geleitet, in dem der Äther zerlegt wurde. Der Reaktor war mit einem Katalysator gefüllt, der durch Absorption von Phosphorsäure auf Kieselgel und anschließendem Erhitzen hergestellt worden war. Die Austrittstemperatur des Reaktionsproduktes aus der Zerlegungsstufe betrug 190°C. Die mittlere Temperatur in der Zerlegungsstufe betrug 180°C. Das Reaktionsprodukt wurde in eine Destillationskolonne geleitet, in der am Kopf das Isobuten und am Sumpf das Isobutanol erhalten wurde, das zur Verätherungsstufe zurückgeführt wurde. Die Ausbeute des in hoher Reinheit erhaltenen Isobutens, bezogen auf das im Beispiel eingesetzte $C_4$-Kohlenwasserstoffgemisch enthaltene Isobuten, betrug 97,6%.

Wurde in der Zerlegungsstufe eine mittlere Temperatur aufrechterhalten, die höher als die Austrittstemperatur des Reaktionsgemisches aus der Zerlegungsstufe lag, so wurden ein Isobutenprodukt mit einer niedrigeren Reinheit und/oder ein Isobutanolsumpfprodukt der Destillationskolonne mit einem erhöhten Gehalt an Isobutyl-tert.-butyläther erhalten.

## Patentansprüche

1. Verfahren zur Gewinnung von Isobuten aus Isobuten enthaltenden $C_4$-Kohlenwasserstoffgemischen durch Umsetzung des Gemisches mit einem primären $C_3$- oder $C_4$-Alkohol in Gegenwart eines Ionenaustauschers in der Wasserstofform, wobei dieser in einem Festbett angeordnet ist, Destillation des bei der Verätherung erhaltenen Reaktionsgemisches, wobei man als Kopfprodukt das $C_4$-Kohlenwasserstoff-Raffinat und als Bodenprodukt den gebildeten tertiären Äther, gegebenenfalls noch mit überschüssigem primärem $C_3$- oder $C_4$-Alkohol abzieht, Zerlegung des Bodenproduktes in einer zweiten, einen sauren Katalysator enthaltenden Reaktionszone bei erhöhter Temperatur in Isobuten und primären $C_3$- oder $C_4$-Alkohol, Destillation des Gemisches aus Isobuten und primärem $C_3$- oder $C_4$-Alkohol, wobei als Kopfprodukt Isobuten und als Bodenprodukt der primäre $C_3$- oder $C_4$-Alkohol abgezogen werden, und Rückführung des erhaltenen primären $C_3$- oder $C_4$-Alkohols in die Reaktionszone für die Verätherung, dadurch gekennzeichnet, daß die Austrittstemperatur des Reaktionsgemisches aus der Verätherungsstufe für die Bildung des tertiären Äthers 3 bis 30°C tiefer liegt als die mittlere Temperatur in der Verätherungsstufe und daß die Austrittstemperatur des Reaktionsgemisches aus der Stufe für die Zerlegung des gebildeten tertiären Äthers 3 bis 30°C höher liegt als die mittlere Temperatur in der Zerlegungsstufe.

2. Verfahren nach Anspruch 1, dadurch gekennzeichnet, daß in der Verätherungsstufe mehrere Reaktionszonen hintereinandergeschaltet werden.

3. Verfahren nach Anspruch 2, dadurch gekennzeichnet, daß ein Teilstrom aus einer Reaktionszone oder je ein Teilstrom aus mehreren oder allen Reaktionszonen abgezweigt und in den Hauptstrom vor einer der vorhergehenden Reaktionszonen zurückgeführt wird.

4. Verfahren nach Ansprüchen 1 bis 3, dadurch gekennzeichnet, daß in der Verätherungsreaktion das im $C_4$-Kohlenwasserstoffgemisch enthaltene Isobuten zu mindestens 90% zum $C_3$- oder $C_4$-Alkyl-tert.-butyläther umgesetzt wird.

## Claims

1. A process for isolating isobutene from $C_4$-hydrocarbon mixtures containing isobutene by reacting the mixture with a primary $C_3$- or $C_4$-alcohol in the presence of an ion exchanger in the hydrogen from which is arranged in a fixed bed; distilling the reaction mixture obtained in the etherification, the $C_4$-hydrocarbon raffinate being withdrawn as top product and the tertiary ether formed, which may or may not contain excess primary $C_3$- or $C_4$-alcohol, being withdrawn as bottom product; decomposing the bottom product in a second reaction zone containing an acid catalyst, at elevated temperature, into isobutene and primary $C_3$- or $C_4$-alcohol; distilling the mixture of isobutene and primary $C_3$- or $C_4$-alcohol, isobutene and the primary $C_3$- or $C_4$-alcohol being withdrawn as top product and bottom product respectively; and recycling the primary $C_3$- or $C_4$-alcohol obtained to the reaction zone for etherification, characterized in that the temperature at which the reaction mixture leaves the etherification stage in which the tertiary ether is formed is 3 to 30°C lower than the mean temperature in the etherification stage, and that the temperature at which the reaction mixture leaves the stage in which the tertiary ether formed is decomposed is 3 to 30°C higher than the mean temperature in the decomposition stage.

2. A process as claimed in claim 1, characterized in that several reaction zones are arranged in series in the etherification stage.

3. A process as claimed in claim 2, characterized in that a bleed stream is taken off one reaction zone or off each of several or all reaction zones and is recycled to the main stream, upstream of one of the preceding reaction zones.

4. A process as claimed in claims 1 to 3, characterized in that the conversion of the isobutene, contained in the $C_4$-hydrocarbon mixture, to the

C$_3$- or C$_4$-alkyl tert.-butyl ether in the etherification reaction is at least 90%.

## Revendications

1. Procédé pour l'obtention d'isobutène à partir de mélanges d'hydrocarbures en C$_4$ contenant de l'isobutène, par réaction du mélange avec un alcool primaire en C$_3$ ou C$_4$ en présence d'un échangeur d'ions sous la forme hydrogène, celui-ci étant disposé en un lit fixe; distillation du mélange réactionnel provenant de l'éthérification avec obtention, en tant que produit de tête, du raffinat d'hydrocarbures en C$_4$ et, en tant que produit de fond, de l'éter tertiaire formé, le cas échéant avec l'alcool primaire en C$_3$ ou C$_4$ introduit en excès; décomposition du produit de fond à température élevée dans une seconde zone de réaction contenant un catalyseur acide, en isobutène et en alcool primaire en C$_3$ ou C$_4$; distillation du mélange d'isobutène et d'alcool primaire en C$_3$ ou C$_4$ avec obtention d'isobutène en tant que produit de tête et de l'alcool primaire en C$_3$ ou C$_4$ en tant que produit de fond; et renvoi de l'alcool primaire en C$_3$ ou C$_4$ obtenu dans la zone de réaction pour l'éthérification, caractérisé en ce que la température du mélange réactionnel à la sortie de l'étage d'éthérification pour la formation de l'éther tertiaire est plus basse, de 3 à 30°C, que la température moyenne de l'étage d'éthérification et que la température du mélange réactionnel à la sortie de l'étage pour la décomposition de l'éther tertiaire formé est plus élevée de 3 à 30°C, que la température moyenne de l'étage de décomposition.

2. Procédé selon la revendication 1, caractérisé en ce que plusieurs zones de réaction sont disposées en série dans l'étage d'éthérification.

3. Procédé selon la revendication 2, caractérisé en ce qu'un courant partiel est dérivé d'une zone de réaction ou des courants partiels respectifs sont dérivés de plusieurs ou de toutes les zones de réaction, pour être ramenés dans le courant principal en amont de l'une des zones de réaction précédentes.

4. Procédé selon l'une quelconque des revendications 1, 2 et 3, caractérisé en ce que dans la réaction d'éthérification, l'isobutène contenu dans le mélange d'hydrocarbures en C$_4$ est transformé, pour au moins 90%, en éther alcoyl (en C$_3$ ou C$_4$)-tertiobutylique.